(19) Europäisches Patentamt / European Patent Office / Office européen des brevets

(11) **EP 3 367 218 A1**

(12) **EUROPEAN PATENT APPLICATION**

(43) Date of publication:
29.08.2018 Bulletin 2018/35

(21) Application number: 18150726.0

(22) Date of filing: 02.05.2005

(51) Int Cl.:
G06F 3/0346 (2013.01)    G06F 3/01 (2006.01)
A61B 5/11 (2006.01)     A61B 5/00 (2006.01)
G06F 21/32 (2013.01)    A61B 5/117 (2016.01)
G06K 9/00 (2006.01)     H04N 5/44 (2011.01)
H04N 21/422 (2011.01)

(84) Designated Contracting States:
AT BE BG CH CY CZ DE DK EE ES FI FR GB GR
HU IE IS IT LI LT LU MC NL PL PT RO SE SI SK TR

(30) Priority: 30.04.2004   US 566444 P
23.09.2004   US 612571 P
05.01.2005   US 641410 P

(62) Document number(s) of the earlier application(s) in
accordance with Art. 76 EPC:
10014911.1 / 2 337 016
05744089.3 / 1 741 088

(71) Applicant: IDHL Holdings, Inc.
Wilmington, DE 19809 (US)

(72) Inventor: LIBERTY, Matthew
POTOMAC, MD Maryland 20854 (US)

(74) Representative: Diehl & Partner GbR
Patentanwälte
Erika-Mann-Strasse 9
80636 München (DE)

Remarks:
•Claims filed after the date of filing of the application
(Rule 68(4) EPC).
•This application was filed on 09-01-2018 as a
divisional application to the application mentioned
under INID code 62.

(54) **FREE SPACE POINTING DEVICE WITH TILT COMPENSATION AND IMPROVED USABILITY**

(57)    A handheld device comprises a three-axis accelerometer (506) for determining an acceleration of the device and outputting an acceleration output associated therewith; a plurality of rotational sensors (502, 504) for determining rotation of the device about a respective plurality of axes and outputting a respective plurality of rotational outputs associated therewith; and a processing unit for: determining movement of the device in the first frame of reference based on the acceleration output and the plurality of rotational outputs; determining an orientation of the device in the first frame of reference based on the angular velocities of the device in the first frame of reference and the acceleration output corresponding to the acceleration of the device due to gravity in the first frame of reference; and generating a rotation quaternion for use with rotating the determined movement from the first frame of reference to a second frame of reference.

FIG. 4

EP 3 367 218 A1

**Description**

RELATED APPLICATIONS

This application is related to, and claims priority from, U.S. Provisional Patent Application Serial No. 60/566,444 filed on April 30, 2004, entitled "Freespace Pointing Device", the disclosure of which is incorporated here by reference. This application is also related to, and claims priority from, U.S. Provisional Patent Application Serial No. 60/612,571, filed on September 23, 2004, entitled "Free Space Pointing Devices and Methods", the disclosure of which is incorporated here by reference. This application is also related to, and claims priority from, U.S. Provisional Patent Application Serial No. 60/641,410, filed on January 5, 2005, entitled "Freespace Pointing Devices and Methods for Using Same", the disclosure of which is incorporated here by reference. This application is also related to U.S. Patent Applications Nos. 11/119,987, 11/119,688, and 11/119,663 , entitled "Methods and Devices for Removing Unintentional Movement in Free Space Pointing Devices", "Methods and Devices for Identifying Users Based on Tremor", and "Free Space Pointing Devices and Methods", all of which were filed concurrently here with and all of which are incorporated here by reference.

BACKGROUND

The present invention relates generally to handheld, pointing devices and, more specifically to free space pointing devices and techniques for tilt compensation and improved usability associated therewith.

Technologies associated with the communication of information have evolved rapidly over the last several decades. Television, cellular telephony, the Internet and optical communication techniques (to name just a few things) combine to inundate consumers with available information and entertainment options. Taking television as an example, the last three decades have seen the introduction of cable television service, satellite television service, pay-per-view movies and video-on-demand. Whereas television viewers of the 1960s could typically receive perhaps four or five over-the-air TV channels on their television sets, today's TV watchers have the opportunity to select from hundreds, thousands, and potentially millions of channels of shows and information. Video-on-demand technology, currently used primarily in hotels and the like, provides the potential for in-home entertainment selection from among thousands of movie titles.

The technological ability to provide so much information and content to end users provides both opportunities and challenges to system designers and service providers. One challenge is that while end users typically prefer having more choices rather than fewer, this preference is counterweighted by their desire that the selection process be both fast and simple. Unfortunately, the development of the systems and interfaces by which end users access media items has resulted in selection processes which are neither fast nor simple. Consider again the example of television programs. When television was in its infancy, determining which program to watch was a relatively simple process primarily due to the small number of choices. One would consult a printed guide which was formatted, for example, as series of columns and rows which showed the correspondence between (1) nearby television channels, (2) programs being transmitted on those channels and (3) date and time. The television was tuned to the desired channel by adjusting a tuner knob and the viewer watched the selected program. Later, remote control devices were introduced that permitted viewers to tune the television from a distance. This addition to the user-television interface created the phenomenon known as "channel surfing" whereby a viewer could rapidly view short segments being broadcast on a number of channels to quickly learn what programs were available at any given time.

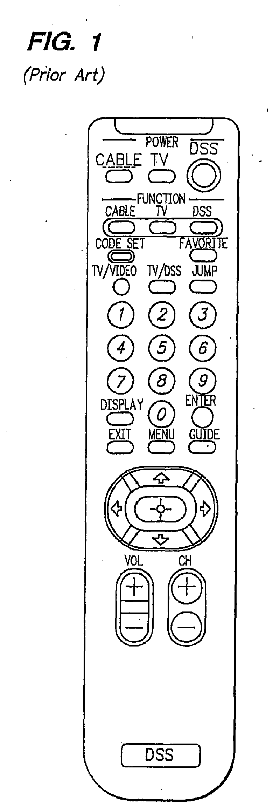

Despite the fact that the number of channels and amount of viewable content has dramatically increased, the generally available user interface, control device options and frameworks for televisions has not changed much over the last 30 years. Printed guides are still the most prevalent mechanism for conveying programming information. The multiple button remote control with up and down arrows is still the most prevalent channel/content selection mechanism. The reaction of those who design and implement the TV user interface to the increase in available media content has been a straightforward extension of the existing selection procedures and interface objects. Thus, the number of rows in the printed guides has been increased to accommodate more channels. The number of buttons on the remote control devices has been increased to support additional functionality and content handling, e.g., as shown in Figure 1. However, this approach has significantly increased both the time required for a viewer to review the available information and the complexity of actions required to implement a selection. Arguably, the cumbersome nature of the existing interface has hampered commercial implementation of some services, e.g., video-on-demand, since consumers are resistant to new services that will add complexity to an interface that they view as already too slow and complex.

In addition to increases in bandwidth and content, the user interface bottleneck problem is being exacerbated by the aggregation of technologies. Consumers are reacting positively to having the option of buying integrated systems rather than a number of segregable components. An example of this trend is the combination television/VCR/DVD in which three previously independent components are frequently sold today as an integrated unit. This trend is likely to continue, potentially with an end result that most if not all of the communication devices currently found in the household will be packaged together as an integrated unit, e.g., a television/VCR/DVD/internet access/radio/stereo unit. Even those who continue to buy separate components will likely desire seamless control of, and interworking between, the separate components. With this increased aggregation comes the potential for more complexity in the user interface. For example, when so-called "universal" remote units were introduced, e.g., to combine the functionality of TV remote units and VCR remote units, the number of buttons on these undiversal remote units was typically more than the number of buttons on either the TV remote unit or VCR remote unit individually. This added number of buttons and functionality makes it very difficult to control anything but the simplest aspects of a TV or VCR without hunting for exactly the right button on the remote. Many times, these universal remotes do not provide enough buttons to access many levels of control or features unique to certain TVs. In these cases, the original device remote unit is still needed, and the original hassle of handling multiple remotes remains due to user interface issues arising from the complexity of aggregation. Some remote units have addressed this problem by adding "soft" buttons that can be programmed with the expert commands. These soft buttons sometimes have accompanying LCD displays to indicate their action. These too have the flaw that they are difficult to use without looking away from the TV to the remote control. Yet another flaw in these remote units is the use of modes in an attempt to reduce the number of buttons. In these "moded" universal remote units, a special button exists to select whether the remote should communicate with the TV, DVD player, cable set-top box, VCR, etc. This causes many usability issues including sending commands to the wrong device, forcing the user to look at the remote to make sure that it is in the right mode, and it does not provide any simplification to the integration of multiple devices. The most advanced of these universal remote units provide some integration by allowing the user to program sequences of commands to multiple devices into the remote. This is such a difficult task that many users hire professional installers to program their universal remote units.

Some attempts have also been made to modernize the screen interface between end users and media systems. However, these attempts typically suffer from, among other drawbacks, an inability to easily scale between large collections of media items and small collections of media items. For example, interfaces which rely on lists of items may work well for small collections of media items, but are tedious to browse for large collections of media items. Interfaces which rely on hierarchical navigation (e.g., tree structures) may be speedier to traverse than list interfaces for large collections of media items, but are not readily adaptable to small collections of media items. Additionally, users tend to lose interest in selection processes wherein the user has to move through three or more layers in a tree structure. For all of these cases, current remote units make this selection processor even more tedious by forcing the user to repeatedly depress the up and down buttons to navigate the list or hierarchies. When selection skipping controls are available such as page up and page down, the user usually has to look at the remote to find these special buttons or be trained to know that they even exist. Accordingly, organizing frameworks, techniques and systems which simplify the control and screen interface between users and media systems as well as accelerate the selection process, while at the same time permitting service providers to take advantage of the increases in available bandwidth to end user equipment by facilitating the supply of a large number of media items and new services to the user have been proposed in U.S. Patent Application Serial No. 10/768,432, filed on January 30,2004, entitled "A Control Framework with a Zoomable Graphical User Interface for Organizing, Selecting and Launching Media Items", the disclosure of which is incorporated here by reference.

Of particular interest for this specification are the remote devices usable to interact with such frameworks, as well as other applications and systems. As mentioned in the above-incorporated application, various different types of remote devices can be used with such frameworks including, for example, trackballs, "mouse"-type pointing devices, light pens, etc. However, another category of remote devices which can be used with such frameworks (and other applications) is free space pointing devices. The phrase "free space pointing" is used in this specification to refer to the ability of an input device to move in three (or more) dimensions in the air in front of, e.g., a display screen, and the corresponding ability of the user interface to translate those motions directly into user interface commands, e.g., movement of a cursor on the display screen. The transfer of data between the free space pointing device may be performed wirelessly or via a wire connecting the free space pointing device to another device. Thus "free space pointing" differs from, e.g., conventional computer mouse pointing techniques which use a surface, e.g., a desk surface or mousepad, as a proxy surface from which relative movement of the mouse is translated into cursor movement on the computer display screen. An example of a free space pointing device can be found in U.S. Patent No. 5,440,326.

The '326 patent describes, among other things, a vertical gyroscope adapted for use as a pointing device for controlling the position of a cursor on the display of a computer. A motor at the core of the gyroscope is suspended by two pairs of orthogonal gimbals from a hand-held controller device and nominally oriented with its spin axis vertical by a pendulous device. Electro-optical shaft angle encoders sense the orientation of a hand-held controller device as it is manipulated by a user and the resulting electrical output is converted into a format usable by a computer to control the movement of a cursor on the screen of the computer display.

However, the freedom of use associated with free-space pointers creates additional challenges. For example, since there is generally no proxy surface on which a free space pointing device rests, the orientation of the handheld control device may vary considerably from user to user or even use to use. If a free space pointing device is used to, for example, control the movement of a cursor displayed on a screen, then some mapping is performed between the detected movement of the handheld device and the movement of then cursor on the screen.

One technique for performing this mapping is to use the body frame of the device as the frame of reference for mapping detected motion of the free space pointing device into intended motion of the cursor. The term "body frame" refers to a set of axes associated with the body of the object being moved as described in more detail below. Using the body frame of reference to perform the mapping, however, has certain drawbacks. For example, it requires the user to hold the device in a certain orientation in order to obtain the cursor movement he or she desires. For example, if the user holds the device on its side and moves the device left to right, the cursor will move vertically, not horizontally, on the screen.

Accordingly, the present invention describes methods and devices for processing the data received from sensor(s) in a manner which addresses these and other problems associated with conventional free space pointing devices.

## SUMMARY

Systems and methods according to the present invention describe free space pointing devices which enhance usability by transforming sensed motion data from a first frame of reference (e.g., the body of the free space pointing device) into a second frame of reference (e.g., a user's frame of reference). One exemplary embodiment of the present invention removes effects associated with a tilt orientation in which the free space pointing device is held by a user.

According to an exemplary embodiment of the present invention, a handheld, pointing device includes a first rotational sensor for determining rotation of the pointing device about a first axis and generating a first rotational output associated therewith, a second rotational sensor for determining rotation of the pointing device about a second axis and generating a second rotational output associated therewith, an accelerometer for determining an acceleration of the pointing device and outputting an acceleration output associated therewith and a processing unit for receiving the first and second rotational outputs and the acceleration output and for: (a) converting the first and second rotational outputs and the acceleration output from a body frame of reference associated with the handheld pointing device into a user's frame of reference in order to remove the effects of tilt associated with the manner in which a user is holding the handheld, pointing device; and (b) determining data associated with x and y coordinates which are in turn associated with movement of a screen cursor, the data based on the converted first and second rotational outputs and the converted acceleration output, wherein the step of converting renders the movement of the screen cursor substantially independent of an orientation in which a user holds the handheld device.

According to another exemplary embodiment of the present invention, a method for using a free space pointing device includes the steps of detecting movement of the free space pointing device and compensating the detected movement by transforming the detected movement from a body frame of reference associated with the free space pointing device into an inertial frame of reference.

According to yet another exemplary embodiment of the present invention, a free space, handheld device includes at least one sensor for detecting movement of the free space pointing device and a processing unit for compensating the detected movement by transforming the detected movement from a body frame of reference associated with the free space pointing device into an inertial frame of reference.

## BRIEF DESCRIPTION OF THE DRAWINGS

The accompanying drawings illustrate exemplary embodiments of the present invention, wherein:

FIG. 1 depicts a conventional remote control unit for an entertainment system;
FIG. 2 depicts an exemplary media system in which exemplary embodiments of the present invention can be implemented;
FIG. 3 shows a free space pointing device according to an exemplary embodiment of the present invention;
FIG. 4 illustrates a cutaway view of the free space pointing device in FIG. 4 including two rotational sensors and one accelerometer;
FIG. 5 is a block diagram illustrating processing of data associated with free space pointing devices according to an exemplary embodiment of the present invention;
FIGS. 6(a) -6(d) illustrate the effects of tilt;
FIG. 7 depicts a hardware architecture of a free space pointing device according to an exemplary embodiment of the present invention;
FIG. 8 is a state diagram depicting a stationary detection mechanism according to an exemplary embodiment of the present invention;
FIG. 9 is a block diagram illustrating transformation of sensed motion data from a first frame of reference into a second frame of reference according to an exemplary embodiment of the present invention; and
FIG. 10 graphically illustrates the transformation of sensed motion data from a first frame of reference into a second frame of reference according to an exemplary embodiment of the present invention

## DETAILED DESCRIPTION

The following detailed description of the invention refers to the accompanying drawings. The same reference numbers in different drawings identify the same or similar elements. Also, the following detailed description does not limit the invention. Instead, the scope of the invention is defined by the appended claims.

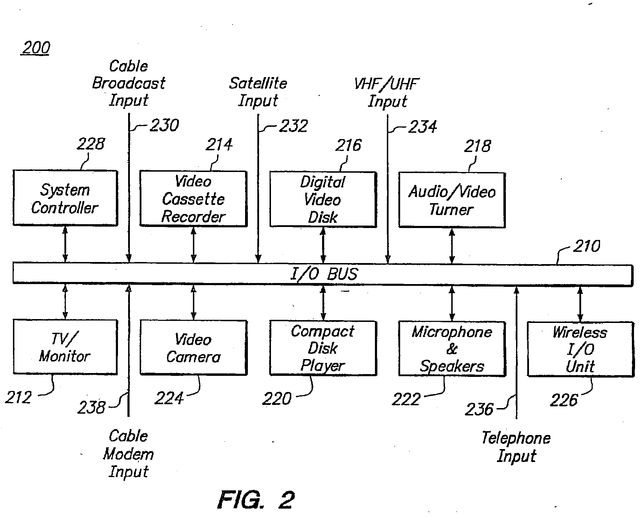

In order to provide some context for this discussion, an exemplary aggregated media system 200 in which the present invention can be implemented will first be described with respect to Figure 2. Those skilled in the art will appreciate, however, that the present invention is not restricted to implementation in this type of media system and that more or fewer components can be included therein. Therein, an input/output (I/O) bus 210 connects the system components in the media system 200 together. The I/O bus 210 represents any of a number of different of mechanisms and techniques for routing signals between the media system components. For example, the I/O bus 210 may include an appropriate number of independent audio "patch" cables that route audio signals, coaxial cables that route video signals, two-wire serial lines or infrared or radio frequency transceivers that route control signals, optical fiber or any other routing mechanisms that route other types of signals.

In this exemplary embodiment, the media system 200 includes a television/monitor 212, a video cassette recorder (VCR) 214, digital video disk (DVD) recorder/playback device 216, audio/video tuner 218 and compact disk player 220 coupled to the I/O bus 210. The VCR 214, DVD 216 and compact disk player 220 may be single disk or single cassette devices, or alternatively may be multiple disk or multiple cassette devices. They may be independent units or integrated together. In addition, the media system 200 includes a microphone/speaker system 222, video camera 224 and a wireless I/O control device 226. According to exemplary embodiments of the present invention, the wireless I/O control device 226 is a free space pointing device according to one of the exemplary embodiments described below. The wireless I/O control device 226 can communicate with the entertainment system 200 using, e.g., an IR or RF transmitter or transceiver. Alternatively, the I/O control device can be connected to the entertainment system 200 via a wire.

The entertainment system 200 also includes a system controller 228. According to one exemplary embodiment of the present invention, the system controller 228 operates to store and display entertainment system data available from a plurality of entertainment system data sources and to control a wide variety of features associated with each of the system components. As shown in Figure 2, system controller 228 is coupled, either directly or indirectly, tp each of the system components, as necessary, through I/O bus 210. In one exemplary embodiment, in addition to or in place of I/O bus 210, system controller 228 is configured with a wireless communication transmitter (or transceiver), which is capable of communicating with the system components via IR signals or RF signals. Regardless of the control medium, the system controller 228 is configured to control the media components of the media system 200 via a graphical user interface described below.

As further illustrated in Figure 2, media system 200 may be configured to receive media items from various media sources and service providers. In this exemplary embodiment, media system 200 receives media input from and, optionally, sends information to, any or all of the following sources: cable broadcast 230, satellite broadcast 232 (e.g., via a satellite dish), very high frequency (VHF) or ultra high frequency (UHF) radio frequency communication of the broadcast television networks 234 (e.g., via an aerial antenna), telephone network 236 and cable modem 238 (or another source of Internet content). Those skilled in the art will appreciate that the media components and media sources illustrated and described with respect to Figure 2 are purely exemplary and that media system 200 may include more or fewer of both. For example, other types of inputs to the system include AM/FM radio and satellite radio.

More details regarding this exemplary entertainment system and frameworks associated therewith can be found in the above-incorporated by reference U.S. Patent Application "A Control Framework with a Zoomable Graphical User Interface for Organizing, Selecting and Launching Media Items". Alternatively, remote devices in accordance with the present invention can be used in conjunction with other systems, for example computer systems including, e.g., a display, a processor and a memory system or with various other systems and applications.

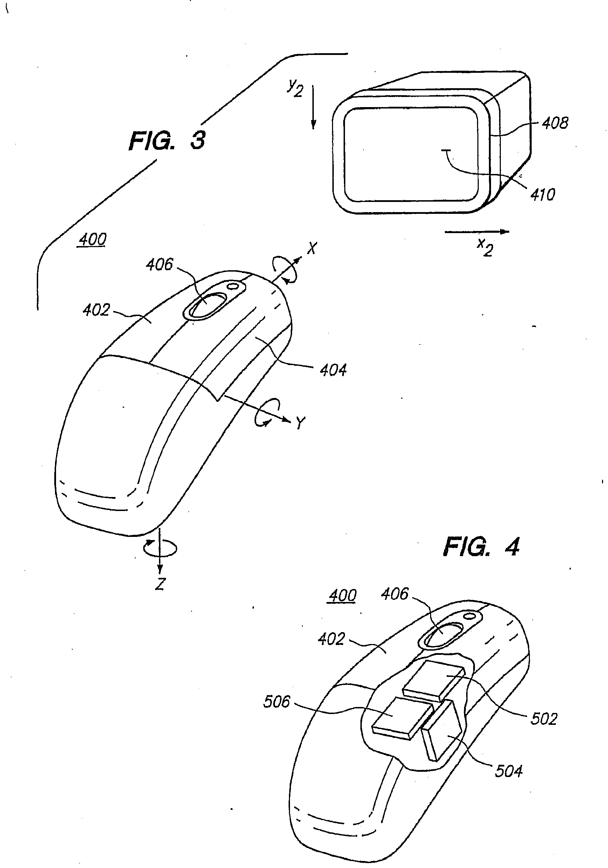

As mentioned in the Background section, remote devices which operate as free space pointers are of particular interest for the present specification. Such devices enable the translation of movement, e.g., gestures, into commands to a user interface. An exemplary free space pointing device 400 is depicted in Figure 3. Therein, user movement of the free space pointing can be defined, for example, in terms of a combination of x-axis attitude (roll), y-axis elevation (pitch) and/or z-axis heading (yaw) motion of the free space pointing device 400. In addition, some exemplary embodiments of the present invention can also measure linear movement of the free space pointing device 400 along the x, y, and z axes to generate cursor movement or other user interface commands. In the exemplary embodiment of Figure 3, the free space pointing device 400 includes two buttons 402 and 404 as well as a scroll wheel 406, although other exemplary embodiments will include other physical configurations. According to exemplary embodiments of the present invention, it is anticipated that free space pointing devices 400 will be held by a user in front of a display 408 and that motion of the free space pointing device 400 will be translated by the free space pointing device into output which is usable to interact with the information displayed on display 408, e.g., to move the cursor 410 on the display 408. For example, rotation of the free space pointing device 400 about the y-axis can be sensed by the free space pointing device 400 and translated into an output usable by the system to move cursor 410 along the $y_2$ axis of the display 408. Likewise, rotation of the free space pointing device 408 about the z-axis can be sensed by the free space pointing device 400 and translated into an output usable by the system to move cursor 410 along the $x_2$ axis of the display 408. It will be appreciated that the output of free space pointing device 400 can be used to interact with the display 408 in a number of ways other than (or in addition to) cursor movement, for example it can control cursor fading, volume or media transport (play, pause, fast-forward and rewind). Input commands may include operations in addition to cursor movement, for example, a zoom in or zoom out on a particular region of a display. A cursor may or may not be visible. Similarly, rotation of the free space pointing device 400 sensed about the x-axis of free space pointing device 400 can be used in addition to, or as an alternative to, y-axis and/or z-axis rotation to provide input to a user interface.

According to one exemplary embodiment of the present invention, two rotational sensors 502 and 504 and one accelerometer 506 can be employed as sensors in free space pointing device 400 as shown in Figure 4. The rotational sensors 502 and 504 can, for example, be implemented using ADXRS150 or ADXRS401 sensors made by Analog Devices. It will be appreciated by those skilled in the art that other types of rotational sensors can be employed as rotational sensors 502 and 504 and that the ADXRS150 and ADXRS401 are purely used as an illustrative example. Unlike traditional gyroscopes, the ADXRS150 rotational sensors use MEMS technology to provide a resonating mass which is attached to a frame so that it can resonate only along one direction. The resonating mass is displaced when the body to which the sensor is affixed is rotated around the sensor's sensing axis. This displacement can be measured using the Coriolis acceleration effect to determine an angular velocity associated with rotation along the sensing axis. If the rotational sensors 502 and 504 have a single sensing axis (as for example the ADXRS150s), then they can be mounted in the free space pointing device 400 such that their sensing axes are aligned with the rotations to be measured. For this exemplary embodiment of the present invention, this means that rotational sensor 504 is mounted such that its sensing axis is parallel to the y-axis and that rotational sensor 502 is mounted such that its sensing axis is parallel to the z-axis as shown in Figure 4. Note, however, that aligning the sensing axes of the rotational sensors 502 and 504 parallel to the desired measurement axes is not required since exemplary embodiments of the present invention also provide techniques for compensating for offset between axes.

One challenge faced in implementing exemplary free space pointing devices 400 in accordance with the present invention is to employ components, e.g., rotational sensors 502 and 504, which are not too costly, while at the same time providing a high degree of correlation between movement of the free space pointing device 400, a user's expectation regarding how the user interface will react to that particular movement of the free space pointing device and actual user interface performance in response to that movement. For example, if the free space pointing device 400 is not moving, the user will likely expect that the cursor ought not to be drifting across the screen. Likewise, if the user rotates the free space pointing device 400 purely around the y-axis, she or he would likely not expect to see the resulting cursor movement on display 408 contain any significant $x_2$ axis component. To achieve these, and other, aspects of exemplary embodiments of the present invention, various measurements and calculations are performed by the handheld device 400 which are used to adjust the outputs of one or more of the sensors 502, 504 and 506 and/or as part of the input used by a processor to determine an appropriate output for the user interface based on the outputs of the sensors 502, 504 and 506. These measurements and calculations are used to compensate for factors which fall broadly into two categories: (1) factors which are intrinsic to the free space pointing device 400, e.g., errors associated with the particular sensors 502, 504 and 506 used in the device 400 or the way in which the sensors are mounted in the device 400 and (2) factors which are not intrinsic to the free space pointing device 400, but are instead associated with the manner in which a user is using the free space pointing device 400, e.g., linear acceleration, tilt and tremor. Exemplary techniques for handling each of these effects are described below.

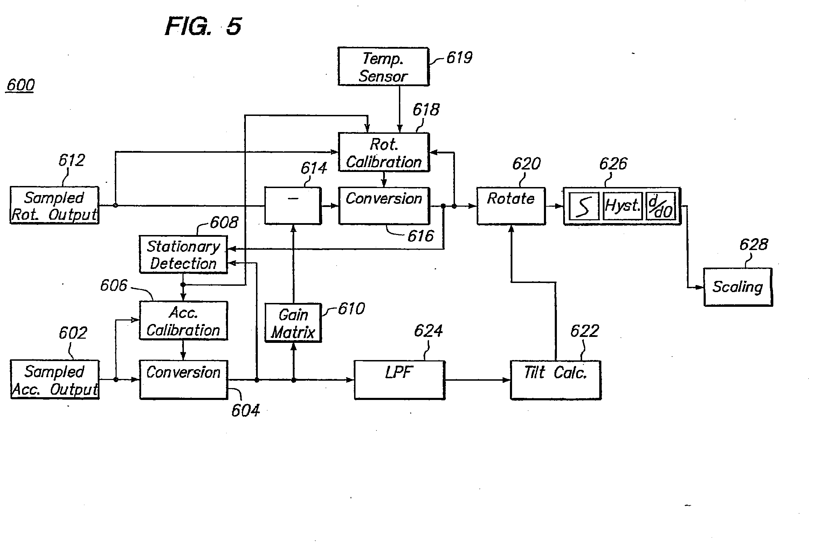

A process model 600 which describes the general operation of free space pointing devices according to exemplary embodiments of the present invention is illustrated in Figure 5. The rotational sensors 502 and 504, as well as the accelerometer 506, produce analog signals which are sampled periodically, e.g., 200 samples/second. For the purposes of this discussion, a set of these inputs shall be referred to using the notation (x, y, z, $\alpha$y, $\alpha$z), wherein x, y, z are the sampled output values of the exemplary three-axis accelerometer 506 which are associated with acceleration of the free space pointing device in the x-axis, y-axis and z-axis directions, respectively, $\alpha$y is a the sampled output value from rotational sensor 502 associated with the rotation of the free space pointing device about the y-axis and az is the sampled output value from rotational sensor 504 associated with rotation of the free space pointing device 400 about the z-axis.

The output from the accelerometer 506 is provided and, if the accelerometer 506 provides analog output, then the output is sampled and digitized by an A/D converter (not shown) to generate sampled accelerometer output 602. The sampled output values are converted from raw units to units of acceleration, e.g., gravities (g), as indicated by conversion function 604. The acceleration calibration block 606 provides the values used for the conversion function 604. This calibration of the accelerometer output 602 can include, for example, compensation for one or more of scale, offset and axis misalignment error associated with the accelerometer 506. Exemplary conversions for the accelerometer data can be performed using the following equation:

$$A = S * ((M-P) .* G(T))$$ (1)

wherein M is a 3x1 column vector composed of the sampled output values (x, y, z), P is a 3x1 column vector of sensor offsets, and S is a 3x3 matrix that contains both scale, axis misalignment, and sensor rotation compensation. G(T) is a gain factor that is a function of temperature. The "*" operator represents matrix multiplication and the ".*" operator represents element multiplication. The exemplary accelerometer 506 has an exemplary full range of +/- 2g. Sensor offset, P, refers to the sensor output, M, for an accelerometer measurement of 0g. Scale refers to the conversion factor between the sampled unit value and g. The actual scale of any given accelerometer sensor may deviate from these nominal scale values due to, e.g., manufacturing variances. Accordingly the scale factor in the equations above will be proportional to this deviation.

Accelerometer 506 scale and offset deviations can be measured by, for example, applying 1g of force along one an axis and measuring the result, R1. Then a -1g force is applied resulting in measurement R2. The individual axis scale, s, and the individual axis offset, p, can be computed as follows:

$$s = (R1 - R2) / 2$$ (2)

$$p = (R1 + R2) / 2$$ (3)

In this simple case, P is the column vector of the p for each axis, and S is the diagonal matrix of the 1/s for each axis.

However, in addition to scale and offset, readings generated by accelerometer 506 may also suffer from cross-axes effects. Cross-axes effects include non-aligned axes, e.g., wherein one or more of the sensing axes of the accelerometer 506 as it is mounted in the free space pointing device 400 are not aligned with the corresponding axis in the inertial frame of reference, or mechanical errors associated with the machining of the accelerometer 506 itself, e.g., wherein even though the axes are properly aligned, a purely y-axis acceleration force may result in a sensor reading along the z-axis of the accelerometer 506. Both of these effects can also be measured and added to the calibration performed by function 606.

The accelerometer 506 serves several purposes in exemplary free space pointing devices according to exemplary embodiments of the present invention. For example, if rotational sensors 502 and 504 are implemented using the exemplary Coriolis effect rotational sensors described above, then the output of the rotational sensors 502 and 504 will vary based on the linear acceleration experienced by each rotational sensor. Thus, one exemplary use of the accelerometer 506 is to compensate for fluctuations in the readings generated by the rotational sensors 502 and 504 which are caused by variances in linear acceleration. This can be accomplished by multiplying the converted accelerometer readings by a gain matrix 610 and subtracting (or adding) the results from (or to) the corresponding sampled rotational sensor data 612. For example, the sampled rotational data $\alpha y$ from rotational sensor 502 can be compensated for linear acceleration at block 614 as:

$$\alpha y' = \alpha y - C * A$$ (4)

wherein C is the 1x3 row vector of rotational sensor susceptibility to linear acceleration along each axis given in units/g and A is the calibrated linear acceleration. Similarly, linear acceleration compensation for the sampled rotational data $\alpha z$ from rotational sensor 504 can be provided at block 614. The gain matrices, C, vary between rotational sensors due to manufacturing differences. C may be computed using the average value for many rotational sensors, or it may be custom computed for each rotational sensor.

Like the accelerometer data, the sampled rotational data 612 is then converted from a sampled unit value into a value associated with a rate of angular rotation, e.g., radians/s, at function 616. This conversion step can also include calibration provided by function 618 to compensate the sampled rotational data for, e.g., scale and offset. Conversion/calibration for both $\alpha y$ and $\alpha z$ can be accomplished using, for example, the following equation:

$$\alpha \, rad/s = (\alpha' - offset(T)) * scale + dOffset$$ (5)

wherein $\alpha'$ refers to the value being converted/calibrated, offset(T) refers to an offset value associated with temperature, scale refers to the conversion factor between the sampled unit value and rad/s, and dOffset refers to a dynamic offset value. Equation (5) may be implemented as a matrix equation in which case all variables are vectors except for scale. In matrix equation form, scale corrects for axis misalignment and rotational offset factors. Each of these variables is discussed in more detail below.

The offset values offset(T) and dOffset can be determined in a number of different ways. When the free space pointing device 400 is not being rotated in, for example, the y-axis direction, the sensor 502 should output its offset value. However, the offset can be highly affected by temperature, so this offset value will likely vary. Offset temperature calibration may be performed at the factory, in which case the value(s) for offset(T) can be preprogrammed into the handheld device 400 or, alternatively, offset temperature calibration may also be learned dynamically during the lifetime of the device. To accomplish dynamic offset compensation, an input from a temperature sensor 619 is used in rotation calibration function 618 to compute the current value for offset(T). The offset(T) parameter removes the majority of offset bias from the sensor readings. However, negating nearly all cursor drift at zero movement can be useful for producing a high-performance pointing device. Therefore, the additional factor dOffset, can be computed dynamically while the free space pointing device 400 is in use. The stationary detection function 608 determines when the handheld is most likely stationary and when the offset should be recomputed. Exemplary techniques for implementing stationary detection function 608, as well as other uses therefore, are described below.

An exemplary implementation of dOffset computation employs calibrated sensor outputs which are low-pass filtered. The stationary output detection function 608 provides an indication to rotation calibration function 618 to trigger computation of, for example, the mean of the low-pass filter output. The stationary output detection function 608 can also control when the newly computed mean is factored into the existing value for dOffset. Those skilled in the art will recognize that a multitude of different techniques can be used for computing the new value for dOffset from the existing value of dOffset and the new mean including, but not limited to, simple averaging, low-pass filtering and Kalman filtering. Additionally, those skilled in the art will recognize that numerous variations for offset compensation of the rotational sensors 502 and 504 can be employed. For example, the offset(T) function can have a constant value (e.g., invariant with temperature), more than two offset compensation values can be used and/or only a single offset value can be computed/used for offset compensation.

After conversion/calibration at block 616, the inputs from the rotational sensors 502 and 504 can be further processed to rotate those inputs into an inertial frame of reference, i.e., to compensate for tilt associated with the manner in which the user is holding the free space pointing device 400, at function 620. Tilt correction is another significant aspect of some exemplary embodiments of the present invention as it is intended to compensate for differences in usage patterns of free space pointing devices according to the present invention. More specifically, tilt correction according to exemplary embodiments of the present invention is intended to compensate for the fact that users will hold pointing devices in their hands at different x-axis rotational positions, but that the sensing axes of the rotational sensors 502 and 504 in the free space pointing devices 400 are fixed. It is desirable that cursor translation across display 408 is substantially insensitive to the way in which the user grips the free space pointing device 400, e.g., rotating the free space pointing device 400 back and forth in a manner generally corresponding to the horizontal dimension ($x_2$-axis) of the display 508 should result in cursor translation along the $x_2$-axis, while rotating the free space pointing device up and down in a manner generally corresponding to the vertical dimension ($y_2$-axis) of the display 508 should result in cursor translation along the $y_2$-axis, regardless of the orientation in which the user is holding the free space pointing device 400.

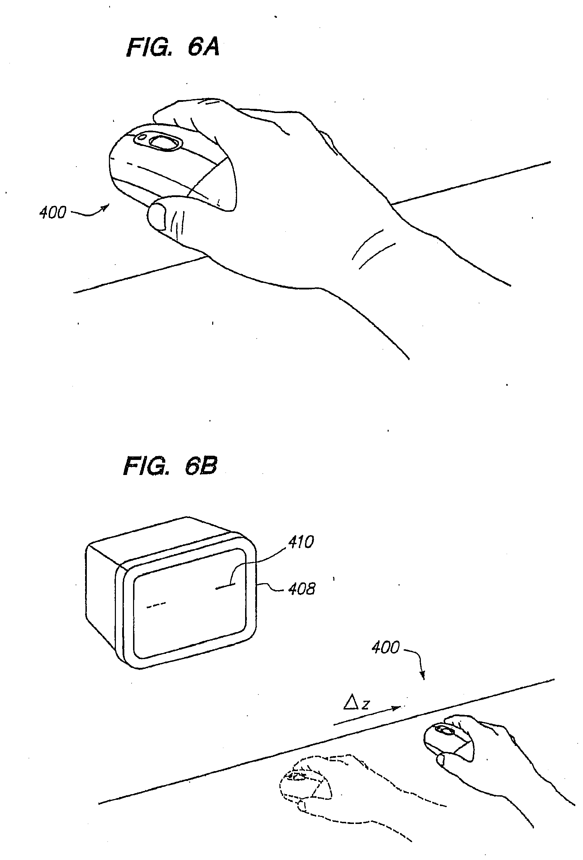

To better understand the need for tilt compensation according to exemplary embodiments of the present invention, consider the example shown in Figure 6(a). Therein, the user is holding free space pointing device 400 in an exemplary inertial frame of reference, which can be defined as having an x-axis rotational value of 0 degrees, e.g., the inertial frame of reference can that in which the free space device has its bottom substantially parallel to a floor of a room in which, e.g., a television is located. The inertial frame of reference can, purely as an example, correspond to the orientation illustrated in Figure 6(a) or it can be defined as any other orientation. Rotation of the free space pointing device 400 in either the y-axis or z-axis directions will be sensed by rotational sensors 502 and 504, respectively. For example, rotation of the free space pointing device 400 around the z-axis by an amount $\Delta z$ as shown in Figure 6(b) will result in a corresponding cursor translation $\Delta x_2$ in the $x_2$ axis dimension across the display 408 (i.e., the distance between the dotted version of cursor 410 and the undotted version).

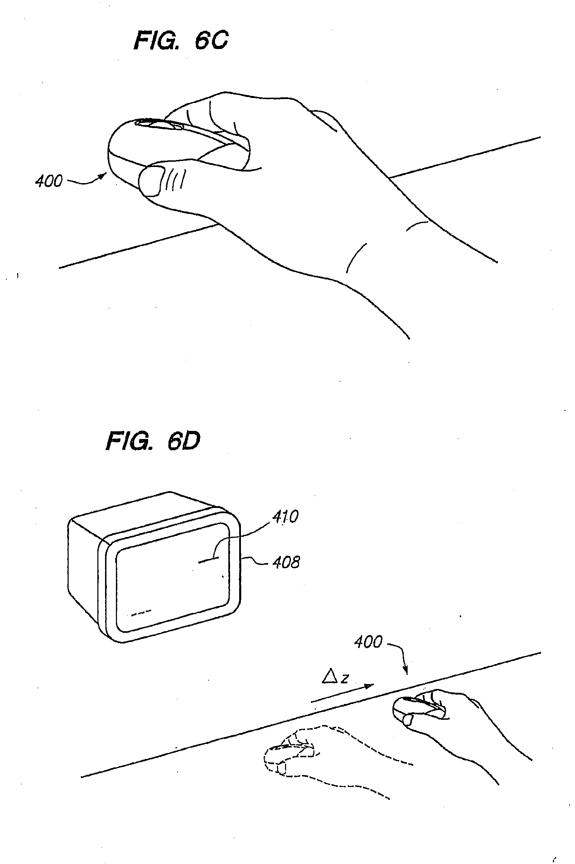

If, on the other hand, the user holds the free space pointing device 400 in a different orientation, e.g., with some amount of x-axis rotation relative to the inertial frame of reference, then the information provided by the sensors 502 and 504 would not (absent tilt compensation) provide an accurate representation of the user's intended interface actions. For example, referring to Figure 6(c), consider a situation wherein the user holds the free space pointing device 400 with an x-axis rotation of 45 degrees relative to the exemplary inertial frame of reference as illustrated in Figure 6(a). Assuming the same z-axis rotation $\Delta z$ is imparted to the free space pointing device 400 by a user as in the example of Figure 6(b), the cursor 410 will instead be translated in both the $x_2$-axis direction and the $y_2$-axis direction as shown in Figure 6(d). This is due to the fact that the sensing axis of rotational sensor 502 is now oriented between the y-axis and the z-axis (because of the orientation of the device in the user's hand). Similarly, the sensing axis of the rotational sensor 504 is also oriented between the y-axis and the z-axis (although in a different quadrant). In order to provide an interface which is transparent to the user in terms of how the free space pointing device 400 is held, tilt compensation according to exemplary embodiments of the present invention translates the readings output from rotational sensors 502 and 504 back into the inertial frame of reference as part of processing the readings from these sensors into information indicative of rotational motion of the free space pointing device 404.

According to exemplary embodiments of the present invention, returning to Figure 5, this can be accomplished by determining the tilt of the free space pointing device 400 using the inputs y and z received from accelerometer 506 at function 622. More specifically, after the acceleration data is converted and calibrated as described above, it can be low pass filtered at LPF 624 to provide an average acceleration (gravity) value to the tilt determination function 622. Then, tilt θ can be calculated in function 622 as:

$$\theta = \tan^{-1}\left(\frac{y}{z}\right) \tag{7}$$

The value θ can be numerically computed as atan2(y,z) to prevent division by zero and give the correct sign. Then, function 620 can perform the rotation R of the converted/calibrated inputs αy and az using the equation:

$$R = \begin{bmatrix} \cos\theta & \sin\theta \\ -\sin\theta & \cos\theta \end{bmatrix} \bullet \begin{bmatrix} \alpha y \\ \alpha z \end{bmatrix} \tag{8}$$

to rotate the converted/calibrated inputs αy and αz to compensate for the tilt θ. Tilt compensation as described in this exemplary embodiment is a subset of a more general technique for translating sensor readings from the body frame of reference into a user's frame of reference according to another exemplary embodiment of the present invention which is described below.

Once the calibrated sensor readings have been compensated for linear acceleration, processed into readings indicative of angular rotation of the free space pointing device 400, and compensated for tilt, post-processing can be performed at blocks 626 and 628. Exemplary post-processing can include compensation for various factors such as human tremor. Although tremor may be removed using several different methods, one way to remove tremor is by using hysteresis. The angular velocity produced by rotation function 620 is integrated to produce an angular position. Hysteresis of a calibrated magnitude is then applied to the angular position. The derivative is taken of the output of the hysteresis block to again yield an angular velocity. The resulting output is then scaled at function 628 (e.g., based on the sampling period) and used to generate a result within the interface, e.g., movement of a cursor 410 on a display 408.

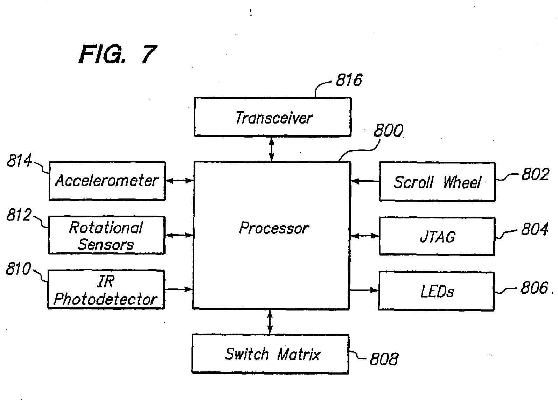

Having provided a process description of exemplary free space pointing devices according to the present invention, Figure 7 illustrates an exemplary hardware architecture. Therein, a processor 800 communicates with other elements of the free space pointing device including a scroll wheel 802, JTAG 804, LEDs 806, switch matrix 808, IR photodetector 810, rotational sensors 812, accelerometer 814 and transceiver 816. The scroll wheel 802 is an optional input component which enables a user to provide input to the interface by rotating the scroll wheel 802 clockwise or counterclockwise. JTAG 804 provides the programming and debugging interface to the processor. LEDs 806 provide visual feedback to a user, for example, when a button is pressed. Switch matrix 808 receives inputs, e.g., indications that a button on the free space pointing device 400 has been depressed or released, that are then passed on to processor 800. The optional IR photodetector 810 can be provided to enable the exemplary free space pointing device to learn IR codes from other remote controls. Rotational sensors 812 provide readings to processor 800 regarding, e.g., the y-axis and z-axis rotation of the free space pointing device as described above. Accelerometer 814 provides readings to processor 800 regarding the linear acceleration of the free space pointing device 400 which can be used as described above, e.g., to perform tilt compensation and to compensate for errors which linear acceleration introduces into the rotational readings generated by rotational sensors 812. Transceiver 816 is used to communicate information to and from free space pointing device 400, e.g., to the system controller 228 or to a processor associated with a computer. The transceiver 816 can be a wireless transceiver, e.g., operating in accordance with the Bluetooth standards for short-range wireless communication or an infrared transceiver. Alternatively, free space pointing device 400 can communicate with systems via a wireline connection.

In the exemplary embodiment of Figure 4, the free space pointing device 400 includes two rotational sensors 502 and 504, as well as an accelerometer 506. However, according to another exemplary embodiment of the present invention, a free space pointing device can alternatively include just one rotational sensor, e.g., for measuring angular velocity in the z-axis direction, and an accelerometer. For such an exemplary embodiment, similar functionality to that described above can be provided by using the accelerometer to determine the angular velocity along the axis which is not sensed by the rotational sensor. For example, rotational velocity around the y-axis can be computed using data generated by the accelerometer and calculating:

$$\omega_Y = \frac{\partial \theta_Y}{\partial t} = \frac{\partial}{\partial t} \tan^{-1}\left(\frac{x}{z}\right) \qquad (9)$$

In addition, the parasitic acceleration effects that are not measured by a rotational sensor should also be removed. These effects include actual linear acceleration, acceleration measured due to rotational velocity and rotational acceleration, and acceleration due to human tremor.

Stationary detection function 608, mentioned briefly above, can operate to determine whether the free space pointing device 400 is, for example, either stationary or active (moving). This categorization can be performed in a number of different ways. One way, according to an exemplary embodiment of the present invention, is to compute the variance of the sampled input data of all inputs (x, y, z, αy, αz) over a predetermined window, e.g., every quarter of a second. This variance is then compared with a threshold to classify the free space pointing device as either stationary or active.

Another stationary detection technique according to exemplary embodiments of the present invention involves transforming the inputs into the frequency domain by, e.g., performing a Fast Fourier Transform (FFT) on the input data. Then, the data can be analyzed using, e.g., peak detection methods, to determine if the free space pointing device 400 is either stationary or active. Additionally, a third category can be distinguished, specifically the case where a user is holding the free space pointing device 400 but is not moving it (also referred to herein as the "stable" state. This third category can be distinguished from stationary (not held) and active by detecting the small movement of the free space pointing device 400 introduced by a user's hand tremor when the free space pointing device 400 is being held by a user. Peak detection can also be used by stationary detection function 608 to make this determination. Peaks within the range of human tremor frequencies, e.g., nominally 8-12 Hz, will typically exceed the noise floor of the device (experienced when the device is stationary and not held) by approximately 20 dB.

In the foregoing examples, the variances in the frequency domain were sensed within a particular frequency range, however the actual frequency range to be monitored and used to characterize the status of the free space pointing device 400 may vary. For example, the nominal tremor frequency range may shift based on e.g., the ergonomics and weight of the free space pointing device 400, e.g., from 8-12 Hz to 4-7 Hz.

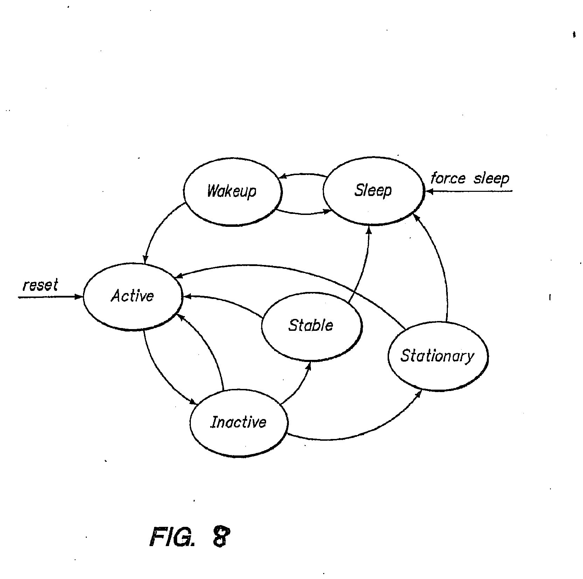

According to another exemplary embodiment of the present invention, stationary detection mechanism 608 can include a state machine. An exemplary state machine is shown in Figure 8. Therein, the ACTIVE state is, in this example, the default state during which the free space pointing device 400 is moving and being used to, e.g., provide inputs to a user interface. The free space pointing device 400 can enter the ACTIVE state on power-up of the device as indicated by the reset input. If the free space pointing device 400 stops moving, it may then enter the INACTIVE state. The various state transitions illustrated in Figure 8 can be triggered by any of a number of different criteria including, but not limited to, data output from one or both of the rotational sensors 502 and 504, data output from the accelerometer 506, time domain data, frequency domain data or any combination thereof. State transition conditions will be generically referred to herein using the convention "Condition$_{state \rightarrow stateB}$". For example, the free space pointing device 400 will transition from the ACTIVE state to the INACTIVE state when condition$_{active \rightarrow inactive}$ occurs. For the sole purpose of illustration, consider that condition$_{active \rightarrow inactive}$ can, in an exemplary free space pointing device 400, occur when mean and/or standard deviation values from both the rotational sensor(s) and the accelerometer fall below first predetermined threshold values for a first predetermined time period.

State transitions can be determined by a number of different conditions based upon the interpreted sensor outputs. Exemplary condition metrics include the variance of the interpreted signals over a time window, the threshold between a reference value and the interpreted signal over a time window, the threshold between a reference value and the filtered interpreted signal over a time window, and the threshold between a reference value and the interpreted signal from a start time can be used to determine state transitions. All, or any combination, of these condition metrics can be used to trigger state transitions. Alternatively, other metrics can also be used. According to one exemplary embodiment of the present invention, a transition from the INACTIVE state to the ACTIVE state occurs either when (1) a mean value of sensor output(s) over a time window is greater than predetermined threshold(s) or (2) a variance of values of sensor output(s) over a time window is greater than predetermined threshold(s) or (3) an instantaneous delta between sensor values is greater than a predetermined threshold.

The INACTIVE state enables the stationary detection mechanism 608 to distinguish between brief pauses during which the free space pointing device 400 is still being used, e.g., on the order of a tenth of a second, and an actual transition to either a stable or stationary condition. This protects against the functions which are performed during the STABLE and STATIONARY states, described below, from inadvertently being performed when the free space pointing device is being used. The free space pointing device 400 will transition back to the ACTIVE state when condition$_{inactive \rightarrow active}$ occurs, e.g., if the free space pointing device 400 starts moving again such that the measured outputs from the rotational sensor(s) and the accelerometer exceeds the first threshold before a second predetermined time period in the INACTIVE state elapses.

The free space pointing device 400 will transition to either the STABLE state. or the STATIONARY state after the second predetermined time period elapses. As mentioned earlier, the STABLE state reflects the characterization of the free space pointing device 400 as being held by a person but being substantially unmoving, while the STATIONARY state reflects a characterization of the free space pointing device as not being held by a person. Thus, an exemplary state machine according to the present invention can provide for a transition to the STABLE state after the second predetermined time period has elapsed if minimal movement associated with hand tremor is present or, otherwise, transition to the STATIONARY state.

The STABLE and STATIONARY states define times during which the free space pointing device 400 can perform various functions. For example, since the STABLE state is intended to reflect times when the user is holding the free space pointing device 400 but is not moving it, the device can record the movement of the free space pointing device 400 when it is in the STABLE state e.g., by storing outputs from the rotational sensor(s) and/or the accelerometer while in this state. These stored measurements can be used to determine a tremor pattern associated with a particular user or users as described below. Likewise, when in the STATIONARY state, the free space pointing device 400 can take readings from the rotational sensors and/or the accelerometer for use in compensating for offset as described above.

If the free space pointing device 400 starts to move while in either the STABLE or STATIONARY state, this can trigger a return to the ACTIVE state. Otherwise, after measurements are taken, the device can transition to the SLEEP state. While in the sleep state, the device can enter a power down mode wherein power consumption of the free space pointing device is reduced and, e.g., the sampling rate of the rotational sensors and/or the accelerometer is also reduced. The SLEEP state can also be entered via an external command so that the user or another device can command the free space pointing device 400 to enter the SLEEP state.

Upon receipt of another command, or if the free space pointing device 400 begins to move, the device can transition from the SLEEP state to the WAKEUP state. Like the INACTIVE state, the WAKEUP state provides an opportunity for the device to confirm that a transition to the ACTIVE state is justified, e.g., that the free space pointing device 400 was not inadvertently jostled.

The conditions for state transitions may be symmetrical or may differ. Thus, the threshold associated with the condition$_{active \rightarrow inactive}$ may be the same as (or different from) the threshold(s) associated with the condition$_{inactive \rightarrow active}$. This enables free space pointing devices according to the present invention to more accurately capture user input. For example, exemplary embodiments which include a state machine implementation allow, among other things, for the threshold for transition into a stationary condition to be different than the threshold for the transition out of a stationary condition.

Entering or leaving a state can be used to trigger other device functions as well. For example, the user interface can be powered up based on a transition from any state to the ACTIVE state. Conversely, the free space pointing device and/or the user interface can be turned off (or enter a sleep mode) when the free space pointing device transitions from ACTIVE or STABLE to STATIONARY or INACTIVE. Alternatively, the cursor 410 can be displayed or removed from the screen based on the transition from or to the stationary state of the free space pointing device 400.

As mentioned above, exemplary embodiments of the present invention process movement data received from sensor(s) in the free space pointing device to convert this data from the frame of reference of the free space pointing device's body into another frame of reference, e.g., the user's frame of reference. In the exemplary application of a free space pointing device used to control a user interface displayed on a screen, e.g., a television, the user's frame of reference might be a coordinate system associated with the television screen. Regardless, translation of the data from the body frame of reference into another frame of reference improves the usability of the handheld device by resulting in an operation that is from the user's perspective rather than the device's perspective. Thus, when the user moves his or her hand from left to right in front of a display while holding the free space pointing device, the cursor will move in the left to right direction regardless of the orientation of the free space pointing device.

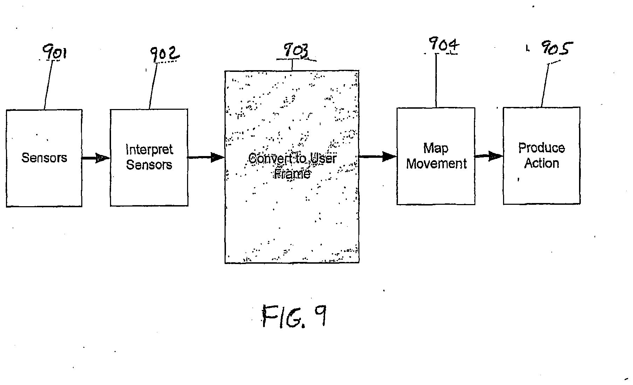

To simplify this discussion an exemplary processing system associated with a free space pointing device is shown in Figure 9, e.g., as described in more detail above. Therein, the handheld system senses motion using one or more sensors 901, e.g., rotational sensor(s), gyroscopes(s), accelerometer(s), magnetometer(s), optical sensor(s), camera(s) or any combination thereof. The sensors are then interpreted in block 902 to produce an estimate of the motion that occurred. The processing block 903 then translates the measured motion from the natural (body) reference frame of the device into the reference frame of the user. The movement is then mapped 904 into meaningful actions that are interpreted at block 905 forwarded to the system to produce a meaningful response, such as moving an on-screen cursor.

Block 903 converts detected movement into the reference frame of the user instead of the reference frame of the device. Orientation may be represented by many different mathematically similar methods including Euler angles, a direction cosine matrix (DCM), or a unit quaternion. Position is generally represented as an offset from the coordinate system origin in a consistent unit including but not limited to meters, centimeters, feet, inches, and miles. In one exemplary embodiment described above, a free space pointing device measures inertial forces including acceleration and rotational velocity. These forces are measured relative to the body of the device by sensors mounted therein. In order to convert the measured data into the user frame of reference, the device estimates both its position and its orientation.

In this exemplary embodiment, it is assumed that the user frame of reference is stationary and has fixed orientation, although those skilled in the art will appreciate that this technique in accordance with the present invention can be readily extended to the cases where the user's frame of reference is non-stationary by either directly transforming to the time-varying frame or by first converting to a stationary frame and then converting to the moving frame. For the stationary, fixed-orientation user frame of reference example, conversion from the body frame to the user frame can be performed by use of the following equations:

$$Pu = Rotate(Pb, Q) + Pdelta$$

$$Pu' = Rotate(Pb', Q)$$

$$Pu'' = Rotate(Pb'', Q)$$

$$Wu = Rotate(Wb, Q)$$

$$Wu' = Rotate(Wb', Q)$$

where:

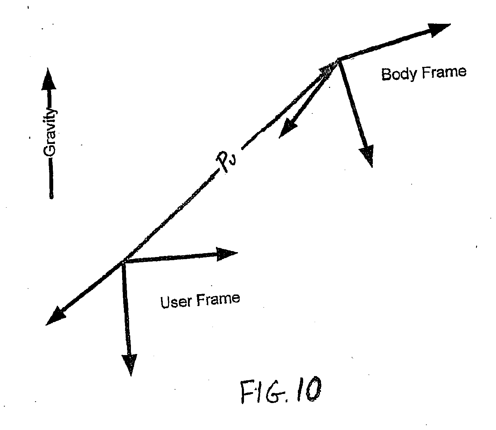

Rotate represents the quaternion rotation operator such that Rotate(A, Q) is equal to Q* A Q where Q* is the quaternion conjugate and the vector A is a quaternion with the complex component equal to A and the real component equal to 0;
Pu is the position in the user frame of reference;
Pb is the position in the device frame of reference;
' represents the derivative. Therefore, Pu' is the derivative of the position in the user frame of reference which is the velocity in the user frame of reference;
Wu is the angular velocity of the device in body angles in the user frame of reference;
Wb is the angular velocity of the device in body angles in the body frame of the device;
Pdelta is the difference between the origin of the user frame of reference and the body frame of reference in the user frame of reference coordinate system;
Q is the normalized rotation quaternion that represents the rotation from the body frame to the user frame. Since the rotation quaternion to rotate from the user frame to the body frame is Q*, we could replace Q with R* where R is the rotation from the user frame to the body frame. Note that Q can be represented in a number of equivalent forms including Euler angles and the direction cosine matrix (DCM), and the above equations may vary slightly in their equivalent forms based upon different representations of Q. Figure 10 graphically illustrates the transformation from a body frame of reference to a user's frame of reference.

During operation, the device estimates Q in an implementation dependent manner to perform this transformation. One exemplary implementation described above involves compensating for tilt (i.e., variations in x-axis roll of the free space pointing device based on the manner in which it is held by a user). The orientation is computed by first estimating the acceleration component due to gravity in the body frame, Ab. By definition, the acceleration vector due to gravity in the user frame, Ag, is set to [0, 0, -1]. Since gravity cannot estimate the heading (rotation about the z-axis), the body frame estimate for heading is used. Therefore, the rotation quaternion has an axis of rotation in the z=0 plane. The following is one of several mathematically equivalent methods for computing the rotation quaternion:

$$V = \|Ab\| \times \|Ag\|$$

(cross product of unit vectors)

$$qV = \|V\|$$

$$\alpha = \sin^{-1}|V|$$

$$Q = \text{Quaternion}[qV, \alpha] = [qV * \sin(\alpha/2), \cos(\alpha/2)]$$

Position is then computed as the double integral of the acceleration in the user frame. The acceleration in the user frame is the acceleration of the body frame rotated into the user frame by Q above. Normally, the origin is assumed to be zero when the device is first activated, but the origin may be reset during normal operation either manually or automatically.

Generally, when the device is not moving, Pu', Pu'', Wu, and Wu'' are all 0. In this exemplary embodiment, Pb'' and Wb are measured. Although an infinite number of rotations Q exist, the minimal rotation can be selected from the available set and used to estimate Wu based on Wb. Alternatively, Q may be computed using an assumed starting offset orientation Qo, by integrating Wb over time as shown using the discrete time integral below:

$$\text{WbAngle} = |Wb| * \text{period}$$

$$Q_{\text{DELTA}} = \text{Quaternion}[Wb, \text{WbAngle}] = [\|Wb\| * \sin(\text{WbAngle}/2), \cos(\text{WbAngle}/2)]$$

$$Q_{\text{NEXT}} = Q_0 {}^{**} Q_{\text{DELTA}}$$

Where * represents multiplication and ** represents quaternion multiplication. Additional stability can be provided by constant field vectors including gravity and the earth's magnetic field and combined with the results above. The combination can be achieved using several numerical and filtering methods including, but not limited to, Kalman filtering.

A variety of different sensors could be employed as long as they measure motion with respect to the body of the device. Exemplary sensors include accelerometers, rotational sensors, gyroscopes, magnetometers and cameras. The user frame does not need to be stationary. For example, if the user's frame of reference is selected to be the user's forearm, then the device would only respond to wrist and finger movement.

One skilled in the art will recognize the commutative property applies to the frame of reference transformations described in this invention. Therefore, the order of the mathematical operations can be altered without materially affecting the invention described herein. In addition, many motion processing algorithms can operate in either frame of reference equivalently, especially when the user frame is chosen to be stationary with a constant orientation.

In addition to providing ease of use, frame of reference transformations according to this exemplary embodiment of the present invention can also be used to address other challenges in handheld device implementations. For example, if a sensor (such as an accelerometer) is not located precisely at the center of rotation in the body frame of reference, the measured acceleration will include both the acceleration of the frame and acceleration components due to the rotation of the frame. Therefore, the measured acceleration can first be transformed to a different target location within the body frame of the device using the following relationship:

$$\text{Abody} = \text{Aaccelerometer} + \omega' \text{ x } R + \omega \text{ x } (\omega \text{ x } R)$$

where R is the vector from the accelerometer to the target location, $\omega$ is the angular velocity of the body frame of reference and $\omega'$ is the angular acceleration of the body frame of reference. If the body frame of the device is constructed such that it lies at R from the accelerometer, then it should have zero angular acceleration effects and may be more easily used to compute the device movement in the user frame. This compensates for intentional or unintentional misalignment between the accelerometer and the center of the body frame of reference. In addition, the estimate of the gravity vector becomes much simpler since there are fewer forces acting at the center of rotation. Then,

$$Auser = Rotate(Abody, Q)$$

where Q is the rotation from the body frame of reference to the accelerometer frame of reference.

Unfortunately, different users have different values for R. For example, one user may use the handheld device by rotating their elbow while another may use the device by rotating their wrist. In addition, people have different sized wrists and forearms. For improved usability this exemplary embodiment of the handheld dynamically computes R and moves the body origin such that it has minimal acceleration components due to angular motion. The exemplary embodiment estimates R by defining R as [Rx, 0, 0] and solving for Rx using and minimizing Abody - Rotate[Ag, Q]. Note that many numerical methods exist including recursive least squares and Kalman filtering that may perform minimization to compute Rx.

Based on the foregoing, it will be appreciated that the present invention describes various techniques for mapping sensed motion of a handheld device from one frame of reference (e.g., a body frame of reference) to another frame of reference (e.g., a user's frame of reference). These mappings can be independent from other mappings associated with the use of the handheld device, e.g., the mapping of sensed motion to cursor movement or can be combined therewith. Moreover, transformations according to the present invention can be performed to transform the sensed motion in all three dimensions, for translational motion and rotational motion or any subset thereof, from the perspective of either the input side of the motion equation or the output side. Additionally, the selection of the frame of reference into which the sensed motion is mapped or transformed can be made in a number of different ways. One example provided above shows the second frame of reference being a user's frame of reference associated with the tilt of the device, however many other variations are possible. For example, the user may select his or her desired frame of reference, which setting can be stored in the handheld as one of a plurality of user preferences and used to perform the transformation. The second frame of reference can be selected based on any number of techniques. The second frame of reference can be selected based upon an explicit command (e.g., button or user interface selection) or automatically through user recognition determined by device use patterns, tremor, and other biometrics.

Additionally, although some of the exemplary embodiments describe above operate on data in the velocity domain, the present invention is not so limited. Mapping or transformation according to the present invention can alternatively or additionally be performed on, for example, position or acceleration data and can be for translational motion, rotational motion or both. Also the order of processing is not critical. For example, if the handheld device is being used to output gesture commands, the mapping can be performed first and then the gesture determined or the gesture can be determined first and then the mapping can be performed.

The above-described exemplary embodiments are intended to be illustrative in all respects, rather than restrictive, of the present invention. Thus the present invention is capable of many variations in detailed implementation that can be derived from the description contained herein by a person skilled in the art. For example, although the foregoing exemplary embodiments describe, among other things, the use of inertial sensors to detect movement of a device, other types of sensors (e.g., ultrasound, magnetic or optical) can be used instead of, or in addition to, inertial sensors in conjunction with the aforedescribed signal processing. All such variations and modifications are considered to be within the scope and spirit of the present invention as defined by the following claims. No element, act, or instruction used in the description of the present application should be construed as critical or essential to the invention unless explicitly described as such. Also, as used herein, the article "a" is intended to include one or more items.

The subject matter set out in the following paragraphs, numbered for convenience, is also disclosed in this application.

(1) A handheld, pointing device comprising: a first rotational sensor for determining rotation of said pointing device about a first axis and generating a first rotational output associated therewith; a second rotational sensor for determining rotation of said pointing device about a second axis and generating a second rotational output associated therewith; an accelerometer for determining an acceleration of said pointing device and outputting an acceleration output associated therewith; and a processing unit for receiving said first and second rotational outputs and said acceleration output and for: (a) converting said first and second rotational outputs and said acceleration output from a body frame of reference associated with said handheld pointing device into a user's frame of reference in order to remove the effects of tilt associated with the manner in which a user is holding said handheld, pointing device; and (b) determining data associated with x and y coordinates which are in turn associated with movement of a screen cursor, said data based on said converted first and second rotational outputs and said converted acceleration output, wherein said step of converting renders said movement of said screen cursor substantially independent of an orientation in which a user holds said handheld device.

(2) The handheld, pointing device of paragraph 1, wherein said user's frame of reference is associated with a television screen.

(3) The handheld, pointing device of paragraph 1, wherein said step of converting further comprises the step of: rotating said first and second rotational outputs into said user's frame of reference by calculating:

$$R = \begin{bmatrix} \cos\theta \sin\theta \\ -\sin\theta \cos\theta \end{bmatrix} \bullet \begin{bmatrix} \alpha y \\ \alpha z \end{bmatrix},$$

wherein $\theta$ is said tilt, $\alpha y$ is said first rotational output and $\alpha z$ is said second rotational output.

(4) A method for using a free space pointing device comprising the steps of: detecting movement of said free space pointing device; and compensating said detected movement by transforming said detected movement from a body frame of reference associated with said free space pointing device into an inertial frame of reference.

(5) The method of paragraph 4, wherein said step of compensating further comprises the steps of: determining a tilt associated with an orientation in which said free space pointing device is held; and compensating said detected movement to correct for said tilt.

(6) The method of paragraph 5, wherein said step of compensating said detected movement to correct for tilt further comprises the step of: rotating first and second rotational outputs into said inertial frame of reference by calculating:

$$R = \begin{bmatrix} \cos\theta \sin\theta \\ -\sin\theta \cos\theta \end{bmatrix} \bullet \begin{bmatrix} \alpha y \\ \alpha z \end{bmatrix},$$

wherein $\theta$ is said tilt, $\alpha y$ is said first rotational output and $\alpha z$ is said second rotational output.

(7) The method of paragraph 4, further comprising the step of: providing information associated with said compensated, detected movement as input to a user interface.

(8) The method of paragraph 5, further comprising the step of: determining said tilt based upon output from an accelerometer.

(9) The method of paragraph 4, wherein said step of detecting movement of said free space pointing device further comprises the step of: detecting said movement using at least one of: a rotational sensor, an accelerometer, a gyroscope, an optical sensor, a magnetometer and a camera.

(10) A free space, handheld device comprising: at least one sensor for detecting movement of said free space pointing device; and a processing unit for compensating said detected movement by transforming said detected movement from a body frame of reference associated with said free space pointing device into an inertial frame of reference.

(11) The free space handheld device of paragraph 10, wherein said processing unit compensates said detected movement by: determining a tilt associated with an orientation in which said free space pointing device is held; and compensating said detected movement to correct for said tilt.

(12) The free space handheld device of paragraph 11, wherein processing unit compensates said detected movement to correct for tilt further by rotating first and second rotational outputs into said inertial frame of reference by calculating:

$$R = \begin{bmatrix} \cos\theta \sin\theta \\ -\sin\theta \cos\theta \end{bmatrix} \bullet \begin{bmatrix} \alpha y \\ \alpha z \end{bmatrix},$$

wherein $\theta$ is said tilt, $\alpha y$ is said first rotational output and $\alpha z$ is said second rotational output.

(13) The free space handheld device of paragraph 10, further comprising: an accelerometer for detecting acceleration of said free space handheld device, wherein an output of said accelerometer is used by said processing unit to determine said tilt.

(14) The free space handheld device of paragraph 10, wherein said at least one sensor includes at least one of: a rotational sensor, an accelerometer, a gyroscope, an optical sensor, a magnetometer and a camera.

(15) A method for operating a free space pointing device comprising the steps of: sensing motion in a body frame of the device; converting said motion into signals representative of motion of the device; transforming the signals from the body frame to another frame of reference; and using the transformed signals to generate an output.

(16) The method of paragraph 15 wherein said step of transforming includes the step of correcting said signals for tilt of said free space pointing device.

(17) The method of paragraph 15, further comprising the step of: calculating at least one intermediate frames of reference between the body frame of reference and the another frame of reference.

(18) The method of paragraph 15, wherein said another frame of reference is a user's frame of reference.

(19) The method of paragraph 15, further comprising the step of: transforming said signals into said body frame of reference from a sensing frame of reference.

(20) The method of paragraph 15, wherein said step of using the transformed signals to generate an output further comprises the step of: producing cursor movement data based on the converted signals.

(21) The method of paragraph 15, wherein the output is a gesture.

(22) A free space pointing device comprising: means for detecting motion of said free space pointing device; and means for compensating said detected motion due to tilt.

(23) A free space pointing device comprising: an accelerometer for measuring an acceleration of said free space pointing device; and a processing unit for determining a center of rotation of said free space pointing device and for determining an acceleration of said free space pointing device at said center of rotation.

(24) The free space pointing device of paragraph 23, wherein said determined acceleration of said free space pointing device at said center of rotation is used by said processing unit to estimate gravity.

(25) The free space pointing device of paragraph 23, wherein the processing unit computes the acceleration due to angular motion by taking the difference between two accelerometers.

(26) The free space pointing device of paragraph 23, wherein the processing unit computes the acceleration due to angular motion by directly measuring the angular velocity of the body.

(27) A method for operating a free space pointing device comprising the steps of: sensing motion of said free space pointing device in a first frame of reference; transforming the sensed motion from the first frame of reference to another frame of reference; and using the transformed signals to generate an output of said free space pointing device.

(28) The method of paragraph 27, further comprising the steps of: converting said sensed motion into signals representative of motion of the free space pointing device; and transforming said signals from said first frame of reference to said another frame of reference.

(29) The method of paragraph 27 wherein said step of transforming includes transforming in x, y, and z dimensions and for both translational and rotational motion.

(30) The method of paragraph 27, wherein said step of transforming includes transforming in a subset of x, y, and z dimensions.

(31) The method of paragraph 27, wherein said step of transforming includes transforming only one of translational motion and rotational motion.

(32) The method of paragraph 27 further comprising the step of: performing a second transformation to generate said output.

(33) The method of paragraph 27, wherein said another frame of reference is selected by a user.

(34) A method for operating a free space pointing device comprising the steps of: sensing motion of said free space pointing device in a first frame of reference; transforming the sensed motion from the first frame of reference to another frame of reference; and identifying a gesture associated with said transformed, sensed motion.

(35) A method for operating a free space pointing device comprising the steps of: sensing motion of said free space pointing device in a first frame of reference; identifying a gesture associated with said sensed motion; and transforming said gesture from a first frame of reference to another frame of reference.

**Claims**

1. A handheld device comprising:

   a three-axis accelerometer for determining an acceleration of the handheld device and outputting an acceleration output associated therewith, wherein the determined acceleration comprises an acceleration of the handheld device due to gravity;
   a plurality of rotational sensors for determining rotation of the handheld device about a respective plurality of axes and outputting a respective plurality of rotational outputs associated therewith, wherein the plurality of rotational outputs comprises angular velocities of the handheld device in a first frame of reference; and
   a processing unit for:

   determining movement of the handheld device in the first frame of reference based on the acceleration output and the plurality of rotational outputs;
   determining an orientation of the handheld device in the first frame of reference based on the angular velocities of the handheld device in the first frame of reference and the acceleration output corresponding to the acceleration of the handheld device due to gravity in the first frame of reference; and
   generating a rotation quaternion for use with rotating the determined movement from the first frame of reference to a second frame of reference, wherein the rotation quaternion is generated using an acceleration vector due to gravity in the second frame of reference and the determined orientation in the first frame of reference, and wherein the first frame of reference is any of a body frame of reference and a device frame of reference.

2. The device of claim 1, wherein the determined movement of the handheld device is a linear acceleration of the handheld device.

3. The device of claim 1, wherein the angular velocities are angular velocities of the handheld device in body angles in the first frame of reference.

4. The device of claim 1, wherein the rotation quaternion is a unit quaternion.

5. The device of claim 1, wherein the processing unit is also for determining a position of the handheld device in the first frame of reference based on the acceleration vector, and wherein the rotation quaternion is generated using the determined position of the handheld device in the first frame, the acceleration vector due to gravity in the second frame of reference and the determined orientation in the first frame of reference.

6. The device of claim 1, wherein the second frame of reference is any of a user frame of reference and an inertial frame of reference.

7. The device of any of the preceding claims, wherein the second frame of reference is any a stationary frame of reference, a non-stationary frame of reference, an intermediary frame of reference and a frame of reference associated with a wrist of a user.

8. The device of any of the preceding claims, wherein the processing unit is also for applying the rotation quaternion to the determined movement.

9. The device of claim 8, wherein the processing unit is also for generating a user interface command using the rotated movement.

10. The device of any of the preceding claims, wherein the acceleration vector is determined by the processing unit using the acceleration output corresponding to the acceleration of the handheld device due to gravity.

11. A method implemented in a handheld device comprising a processing unit, a three-axis accelerometer and a plurality of rotational sensors, the method comprising:

detecting movement of the handheld device using at least one of the three-axis accelerometer and the plurality of rotational sensors;
obtaining, at the processing unit in connection with the detected movement, a plurality of rotational outputs from the plurality of rotational sensors, wherein the plurality of rotational outputs is associated with rotation of the handheld device about a respective plurality of axes, and wherein the plurality of rotational outputs comprises angular velocities of the handheld device in a first frame of reference;
obtaining, at the processing unit in connection with the detected movement, an acceleration output from the three-axis accelerometer, wherein the acceleration output is associated with an acceleration of the handheld device, and wherein the acceleration comprises an acceleration of the handheld device due to gravity;
determining, at the processing unit, the movement of the handheld device in a first frame of reference based on the acceleration output and the plurality of rotational outputs;
determining, at the processing unit, an orientation of the handheld device in the first frame of reference based on the acceleration output corresponding to the acceleration of the handheld device due to gravity in the first frame of reference and the angular velocities of the handheld device in the first frame of reference; and
generating, at the processing unit, a rotation quaternion for use with rotating the determined movement from the first frame of reference to a second frame of reference, wherein the rotation quaternion is generated using an acceleration vector due to gravity in the second frame of reference and the determined orientation in the first frame of reference, and wherein the first frame of reference is any of a body frame of reference and a device frame of reference.

12. The method of claim 11, wherein the determined movement of the handheld device is a linear acceleration of the handheld device.

13. The method of any of the claims 11-12, wherein the second frame of reference is any of a user frame of reference and an inertial frame of reference.

14. The method of any of the claims 11-13, further comprising applying the rotation quaternion to the determined movement.

15. The method of claim 14, further comprising using the rotated movement to generate a user interface command.

# FIG. 1

*(Prior Art)*

200

Cable Broadcast Input 230

Satellite Input 232

VHF/UHF Input 234

228 System Controller

214 Video Cassette Recorder

216 Digital Video Disk

218 Audio/Video Turner

210

I/O BUS

212 TV/Monitor

224 Video Camera

220 Compact Disk Player

222 Microphone & Speakers

226 Wireless I/O Unit

238 Cable Modem Input

236 Telephone Input

**FIG. 2**

FIG. 3

FIG. 4

FIG. 5

FIG. 6A

400

FIG. 6B

410

408

400

$\triangle z$

## FIG. 6C

## FIG. 6D

## FIG. 7

Transceiver — 816

800

814 — Accelerometer

812 — Rotational Sensors

810 — IR Photodetector

Processor

Scroll Wheel — 802

JTAG — 804

LEDs — 806

Switch Matrix — 808

FIG. 8

901

902

903

904

905

| Sensors | → | Interpret Sensors | → | Convert to User Frame | → | Map Movement | → | Produce Action |

FIG. 9

FIG. 10

Europäisches
Patentamt

European
Patent Office

Office européen
des brevets

# EUROPEAN SEARCH REPORT

Application Number

EP 18 15 0726

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 598 187 A (IDE YUJI [JP]; TAKASHIMA KAZUHIRO [JP]; MOROHOSHI TOSHIHIRO [JP]; FUKU) 28 January 1997 (1997-01-28)<br>* column 1, lines 7-15 *<br>* column 6, line 1 - column 12, line 47; figures 1-13 *<br>* column 20, line 43 - column 21, line 60; figures 28-32 * | 1-15 | INV.<br>G06F3/0346<br>G06F3/01<br>A61B5/11<br>A61B5/00<br>G06F21/32<br>A61B5/117<br>G06K9/00<br>H04N5/44<br>H04N21/422 |
| X | US 5 902 968 A (SATO YASUHIRO [JP] ET AL) 11 May 1999 (1999-05-11)<br>* abstract *<br>* column 1, lines 9-11 *<br>* column 4, line 40 - column 5, line 51 *<br>* column 8, lines 55-59 *<br>* column 10, lines 45-50 *<br>* column 15, lines 24-28 *<br>* column 16, lines 24-54 *<br>* figures 1,2,15,34 * | 1-15 | |

TECHNICAL FIELDS
SEARCHED (IPC)

G06F

The present search report has been drawn up for all claims

| Place of search | Date of completion of the search | Examiner |
|---|---|---|
| Munich | 29 May 2018 | Charcos Lloréns, V |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 15 0726

This annex lists the patent family members relating to the patent documents cited in the above-mentioned European search report.
The members are as contained in the European Patent Office EDP file on
The European Patent Office is in no way liable for these particulars which are merely given for the purpose of information.

29-05-2018

| Patent document cited in search report | | Publication date | Patent family member(s) | | Publication date |
|---|---|---|---|---|---|
| US 5598187 | A | 28-01-1997 | NONE | | |
| US 5902968 | A | 11-05-1999 | US | 5902968 A | 11-05-1999 |
| | | | US | 5981884 A | 09-11-1999 |
| | | | US | 6084577 A | 04-07-2000 |
| | | | US | 6229102 B1 | 08-05-2001 |

EPO FORM P0459

For more details about this annex : see Official Journal of the European Patent Office, No. 12/82

REFERENCES CITED IN THE DESCRIPTION

**Patent documents cited in the description**

- US 56644404 P
- US 61257104 P
- US 64141005 P
- US 11119987 A
- US 11119688 B
- US 11119663 B
- US 76843204 A
- US 5440326 A